# EUROPEAN PATENT SPECIFICATION

(11) **EP 1 628 133 B1**
(45) Date of publication and mention of the grant of the patent: **04.03.2009**
(21) Application number: 05018061.1
(22) Date of filing: 19.08.2005
(51) Int. Cl.: G01N 33/00, G01N 31/00

(54) **Concentration monitor of fluid samples containing nitrogen compounds**
Konzentrations-Überwachungsvorrichtung für Stickstoffverbindungen enthaltende Flüssigproben
Dispositif de mesure de la concentration d'échantillons fluides comprenant des composés azotés

(30) Priority: 20.08.2004 JP 2004241304
(43) Date of publication of application: 22.02.2006
(73) Proprietor: HORIBA, LTD., Kyoto-shi, Kyoto 601-8510 (JP)
(72) Inventor: Nakatani, Shigeru, Minami-ku Kyoto-shi Kyoto 601-8510 (JP); Yoshimura, Tomoshi, Minami-ku Kyoto-shi Kyoto 601-8510 (JP); Mori, Yuichi, Minami-ku Kyoto-shi Kyoto 601-8510 (JP)
(74) Representative: Müller - Hoffmann & Partner

(56) References cited:
- EP-A- 1 405 989
- WO-A-99/57558
- JP-A- 55 031 953
- US-A- 3 877 875
- US-A- 3 977 836
- US-A- 4 432 939
- PATENT ABSTRACTS OF JAPAN vol. 004, no. 074 (P-013), 30 May 1980 (1980-05-30) & JP 55 040999 A (SHIMADZU CORP), 22 March 1980 (1980-03-22)

## Description

### BACKGROUND OF THE INVENTION

### 1. Field of the Invention

The invention relates to a nitrogen compound analyzer and specifically relates to a nitrogen compound analyzer for high speed analysis such as a device for analyzing ammonia or nitrogen oxides in automobile exhaust gas.

### 2. Description of the Related Art

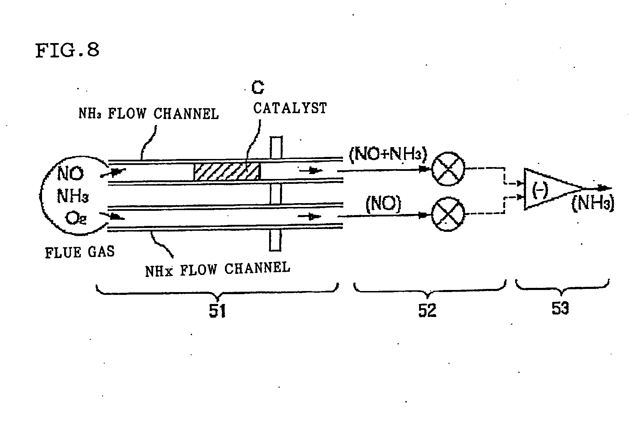

Nitrogen oxides (NOX) and ammonia (NH3) have been key measuring components in exhaust gas from stationary exhaust sources such as gas ducts or from mobile exhaust sources such as automobiles. However, it has been particularly difficult to directly measure nitrogen dioxide (NO2) or NH3. Known means for measuring NH3 includes an NH3 analyzer for stationary exhaust sources which generally has the configuration as shown in Fig. 8 (for example, see Japanese Patent Laid-Open Publication No. 2000-9603). The NH3 analyzer comprises a sampling probe part 51 including two sampling probe lines (an NH3 line and a nitrogen oxide line), an analysis part 52 including analyzers for measuring the NO concentrations of the respective lines, and an arithmetic unit 53 for calculating an NH3 concentration from the NO concentrations determined with the two analyzers. One (the NH3 line) of the sampling probe lines has an oxidation or reduction catalyst C, which may be an ammonia oxidation catalyst or an ammonia reduction catalyst. As used herein, the term "NOX" generally encompasses NO and NO2.

According to the principle of the oxidation type measurement, NH3 in the NH3 line is oxidized to an equimolar amount of NO by the action of the oxidation catalyst, and the concentration of the NO is detected and used to determine the concentration of NH3. Since also NOX generally exists in exhaust gas, NO2 in the exhaust gas is reduced to NO by means of an NO2-NO converter upstream from the detector. In the NOX flow line, only the concentration of NOX is detected, while the total concentration of NH3 and NOX is detected in the NH3 flow line. Thus, the difference between the values detected by the detectors in the two flow lines provides the concentration of NH3. According to the principle of the reduction type measurement, NH3 in the NH3 line is allowed to react with an equimolar amount of NOX by the action of the reduction catalyst, and the concentration of the NOX consumed by the reaction is detected so that the concentration of NH3 is determined. In this case, a reduction in the NOX concentration corresponding to the concentration of NH3 is detected in the NH3 flow line, and the difference between the values detected in both flow lines provides the NH3 concentration.

Concerning mobile exhaust sources, there has been no automobile exhaust gas NH3 analyzer in commercial use, because exhaust gas contains little NH3 during normal driving, NH3 is unstable and capable of being strongly adsorbed, and there has been no measurement means capable of responding to abrupt variations in the exhaust gas component to be measured.

NO2 is also capable of being strongly adsorbed, unstable and highly water-soluble, and thus it is very difficult to directly measure NO2 in exhaust gas. Therefore, a sample is divided as mentioned above, and one part is reduced to NO by means of the NO2-NO converter for measurement of the NOX value, while the other part is directly subjected to measurement of only NO, so that the difference is used to calculate the NO2 value in the method.

However, there has been an increased demand for a device for analyzing NH3 and NOX in automobile exhaust gas that can respond to abrupt variations in the exhaust gas components to be measured and can provide continuous measurement, because environmental analyses have recently become important and various improvements have been made to automobiles or engines. Specifically, there has been a demand for an analyzer capable of providing continuous measurement of the total concentration of NOX and the concentration of each component.

In gasoline engines, for example, tens to thousands of ppm of NH3 can be produced under combustion conditions in a reducing atmosphere called lean-burn or rich spike. Concerning Diesel engines, addition of urea in exhaust gas treatment has recently received attention. Specifically, selective reduction catalysts (SCR) are promising for a method to reduce the amount of NOX in large Diesel automobiles. In this method using an aqueous solution of urea as a reducing agent, NOX discharged from an engine is reduced to harmless nitrogen (N2) on the catalyst. In this process, tens to thousands of ppm of unreacted NH3 can be discharged. Accurate and highly-responsive measurement of such unreacted NH3 is an issue for analyzers. Accurate and highly-responsive measurement of coexisting NOX, NO2 or NO is also an issue for analyzers.

Conventional measurement of the two components NH3 and NO2 in a single sample requires the use of independent analyzers. The conventional measurement of a single sample involves different structures of sample flow lines or treatment means or different interference influence properties or different speeds of response of the analyzers. In the conventional method, therefore, a simple comparison between both measurement data has a precision problem. Easy and accurate measurement of the two components NH3 and NO2 in a single sample is a priority issue.

Particularly when the generated NH3 is converted by oxidation or reduction treatment, the influence of response delay or efficiency reduction (loss during treatment) due to the instability or strong adsorption of NH3 as mentioned above is not negligible. Thus, improvements not only in treatment function in the oxidation means or the reduction means but also in stable responsiveness are urgent issues (hereinafter, these means and any other means for exerting a certain chemical action on a sample refer to as "treatment means".)

Document JP 55 031953 A discloses a measuring method of ammonia in gas which is adapted for performing continuously a measurement of dilute ammonia with a good accuracy by using platinum on a honeycomb carrier for a catalyser with specified conditions. The amount of ammonia is obtained through a measurement of NO based on a catalyzing process using a conversion catalyseur. Ammonia is converted into NO. the amount of NO is measured with an analyzer, and the amount of ammonia contained is thereby continuously obtained.

Document US 4,432,939 A refers to an ammonia gas analyzer. The proposed ammonia gas analyzer includes a gas sampling means, a gas measuring channel connected to the gas sampling means, a comparison gas channel connected parallel to the measuring gas channel with the measuring gas channel including an ammonia to NO converter for converting ammonia in the sample gas into NO, and means for measuring the concentration of ammonia on the basis of variations of an amount of NO in the measuring gas channel with respect to that in the comparison gas channel.

### SUMMARY OF THE INVENTION

It is therefore an object of the invention to provide a nitrogen compound analyzer capable of easily and accurately measuring the two components NH3 and NO2 or each nitrogen compound component in a single sample. More particularly, it is another object of the invention to provide a high-speed-response type nitrogen compound analyzer such as an automobile exhaust gas measuring device, which can quickly follow changes of components in samples.

The object underlying the present invention is achieved by a nitrogen compound analyzer according to independent claim 1. Preferred embodiments of the nitrogen compound analyzer according to the present invention are within the scope of the respective dependent claims.

The inventor has made active investigations and finally found that the nitrogen compound analyzer as described below can fulfill the above objects in completing the invention. The invention is directed to a nitrogen compound analyzer, comprising: a plurality of sample treatment systems by which a single sample is divided, the plurality of sample treatment systems including (A) a treatment system having oxidation means for oxidizing ammonia in the sample and conversion means for converting nitrogen dioxide in the sample to nitrogen monoxide, (B) a treatment system having conversion means for converting nitrogen dioxide in the sample to nitrogen monoxide, and (C) a treatment system in which no conversion treatment is performed on a specific component in the sample; a changeover means capable of providing a specific combination of any of the treatment systems (A), (B) and (C); and at least one measurement means for continuously measuring nitrogen monoxide in the sample.

It is preferred that in order to evaluate the results of continuous analysis of different nitrogen compounds at the same level, the analyzers should be based on a single principle for measurement. According to the invention, a simple and high-precision analyzer capable of quickly following changes in sample components can be provided using at least one measurement means based on a single principle, specifically at least one NO analyzer, and three treatment systems between which comparison, switching and combination can be performed.

Specifically, the components as shown below can be measured using the combination of (A) a treatment system that converts all nitrogen compounds in a sample to NO by oxidizing NH3 to NO and NO2 and converting the NO2 and NO2 part of the sample to NO, (B) a treatment system that converts NOX in the sample to NO by converting the NO2 in the sample to NO, and (C) a treatment system that introduces NO in the sample.
(1) All nitrogen compounds can be measured by the measurement in the treatment system (A).
(2) NOX can be measured by the measurement in the treatment system (B).
(3) NO can be measured by the measurement in the treatment system (C).
(4) NH3 can be measured by the measurement of the difference between the treatment systems (A) and (B).
(5) NO2 can be measured by the measurement of the difference between the treatment systems (B) and (C).

According to the invention, the nitrogen compound analyzer may have a response time information specific to the combination of the measurement means and each of the treatment systems (A), (B) and (C) and may further include an arithmetic processing means for correcting the output of the measurement means with the information.

In the measurement of different components in a sample, the same response time should preferably be provided with respect to each component. Particularly when a difference is determined between the measurements in different treatment systems, such a condition should be more strongly desired from the view of precision. Examples of response time-determining factors for each component include the responsiveness of the gas system and the signal processing system in the measurement means, and the responsiveness of a gas system in the sampling system, specifically a difference in flow rate, and the reactivity or adsorption in the treatment means such as the oxidation means. Since the response time is determined depending on such factors, a correction should be made for the response in each treatment system. Specifically, response time information may be preliminarily obtained for each treatment system, and the output of the measurement means may be corrected using the information during actual measurement. Using such output processing, each nitrogen compound component in a single sample can be easily and accurately measured.

As used herein, the term "response time" refers to the time from switching to a gas of a certain concentration to the time when a specific value with respect to the indicated final value (for example, 90% of the indicated final value according to JIS B 7982 or the like) is achieved, as defined by JIS B 7982 or the like. For evaluation, the response time is generally divided into: time lag Td which is the time until the sample reaches measurement means and allows an analyzer to start an output after switching; and TX which is the time until X% of the indicated final value is achieved from the starting time. In the description, T90 is used as TX, which is the time until 90% of the indicated final value is achieved, similarly to the JIS definition.

The term "arithmetic processing means" refers to a variety of means for processing information about treatments in the analyzer including not only means for making a correction for the response time but also means for processing the signals of the measurement means (including "moving average processing means" as described later) and means for controlling changeover means as described later.

The invention is also directed to a nitrogen compound analyzer, comprising: a plurality of sample treatment systems by which a single sample is divided; at least one measurement means for continuously measuring nitrogen monoxide in the sample; and a changeover means capable of switching flow channels for introducing the sample to oxidation means for oxidizing ammonia and for bypassing the oxidation means, or a changeover means capable of switching flow channels for introducing the sample to conversion means for converting nitrogen dioxide to nitrogen monoxide and for bypassing the conversion means, wherein at least one of total nitrogen compounds, nitrogen oxides, nitrogen monoxide, ammonia, and nitrogen dioxide can be measured.

As mentioned above, it is contemplated that a plurality of fixed treatment systems (for example, three treatment systems (A), (B) and (C)) may be included for performing different treatments. According to the invention, a plurality of treatment means may be switched to form different treatment modes so that the treatment systems can be reduced in number and size, and thus an analyzer can be provided which can easily and accurately measure five nitrogen compound components through two branch channels. Specifically, flow channels and bypass channels may be freely switched for introduction to or bypass of oxidation means for oxidizing NH3 and conversion means for converting NO2 in the sample to NO, so that three treatment modes corresponding to the above treatment systems (A), (B) and (C) can be provided. It will be understood that not all of the treatment modes have to be used and that any of the treatment modes may be selected depending on the component to be measured.

According to the invention, the nitrogen compound analyzer may have a response time information specific to the combination of the measurement means and each of the switched bypass channel and the switched introduction channel with the oxidation means or the conversion means, and may further include an arithmetic processing means for correcting the output of the measurement means with the information.

As mentioned above, the correction may be made for response in each treatment means system. Similarly, a correction should be made for response in the case that treatment means are switched. Specifically, response time information is preliminarily obtained for each treatment mode associated with the changeover in the treatment means, and the output of the measurement means is corrected using the information during actual measurement. This output processing allows simple and accurate measurement of each nitrogen compound component in a single sample.

According to the invention, the nitrogen compound analyzer may further include a signal processing means for receiving and processing the output of the measurement means and outputting or displaying the result, wherein the signal processing means includes a moving average processing means for calculating a moving average of the output.

In the invention as stated above, the correction for response in each treatment system or each treatment mode plays an important role for measurement precision. The inventor has found that if a moving average processing technique is used in the signal correction, the accuracy of the correction can be extremely improved. In particular, this technique is very effective in measuring components capable of being strongly adsorbed such as NH3 and NO2. Specifically, a preferred method includes the steps of: preliminarily obtaining the amount of shift for moving average corresponding to Td in each treatment mode, an output-sampling time corresponding to T90, and a parameter for use in calculation (hereinafter referred to as "an arithmetic parameter"); and correcting actual measurements first for Td and then for T90 to produce a moving average.

In a preferred mode, the output-sampling time or the arithmetic parameter for moving average (hereinafter referred to as "moving average mode") may be freely set depending on the sample condition. Specifically, in the case of measurement of components in automobile exhaust gas, the amounts of discharged NH3 or NO2 may significantly differ between a Diesel engine and a gasoline engine, but even with the same sample treatment system, the accuracy of the measurement can be improved by changing the moving average mode. It may also be useful to switch moving average modes depending on the selected concentration range for measurement. In some cases, moving average modes may also be switched depending on the actual measurement.

In the nitrogen compound analyzer according to the invention, the oxidation means or the conversion means comprises: an inner flow channel; at least one projection portion or step portion provided in the flow channel; and a flat inner surface region of a specific length, which is parallel to the centerline of the flow channel and provided upstream and downstream of the projection or step portion in the flow channel.

As mentioned above, the treatment means according to the invention should have high-speed responsiveness to the compounds capable of being strongly adsorbed such as NH3 and NO2. The NH3-oxidizing means should also quickly produce high temperature conditions of at least 800°C. The inventor has found that a laminar flow is formed and then turned into a turbulent flow by means of a projection portion or a step portion provided downstream of the flow channel, the boundary layer gas and the gas outside the layer are mixed and shifted, the gas shifted to the boundary layer is quickly heated in a flow channel that is provided downstream of the projection portion and parallel to the gas flow (a parallel and flat-shaped portion), and the whole of the gas flow can be heated in a short time, so that the above requirements can be satisfied. When the flow rate is high, a plurality of projections or step portions may be provided so that a quick heating effect can be produced depending on the gas conditions.

Such a mechanism can provide very high performance for the analyzer requiring high-speed response. Particularly in the case that the sampling flow channel is divided into plural parts in which a part of a sample is subjected to specific treatment and compared with untreated part of the sample for measurement, the response lag between the flow channels can be extremely reduced by means of the above mechanism so that real-time measurement can be achieved.

Thus, the two components NH3 and NO2 or each nitrogen compound component in a single sample can be easily and accurately measured using the nitrogen compound analyzer according to the invention. In particular, if a single sample is measured using different treatment systems or using changeover in treatment modes, a significant problem of a response lag between the flow channels can be caused. Against this problem, there can be provided a nitrogen compound analyzer that can make a proper correction depending on the sample condition and can quickly follow changes in components of the sample with quick treatment means. Particularly in the field where there has conventionally been no practically useful product in terms of responsiveness, there can be provided a very useful device such as a device for analyzing NH3 in automobile exhaust gas.

### BRIEF DESCRIPTION OF THE DRAWINGS

Fig. 1 is a diagram showing the basic analyzer configuration according to the invention;
Fig. 2(A) is a diagram schematically showing the responsiveness of an analysis system in the treatment system according to the invention;
Fig. 2(B) is a diagram schematically showing the responsiveness of an analysis system in the treatment system according to the invention;
Fig. 2(C) is a diagram schematically showing the responsiveness of an analysis system in the treatment system according to the invention;
Fig. 2(D) is a diagram schematically showing the responsiveness of an analysis system in the treatment system according to the invention;
Fig. 3 is a diagram showing the basic structure of a catalyst unit;
Fig. 4 is a diagram showing another example of the analyzer configuration according to the invention;
Fig. 5 is a diagram showing a mode of the example of the analyzer configuration according to the invention;
Fig. 6 is a diagram showing another mode of the example of the analyzer configuration according to the invention;
Fig. 7 is a diagram showing a third example of the analyzer configuration according to the invention; and
Fig. 8 is a diagram schematically showing the configuration of an analyzer according to a conventional technique.

In the drawings, reference numeral 1 represents a heating part, 2, 2a and 5 a tubular part, 3 a projection portion, 4 a parallel and flat-shaped part, 6 a catalyst, 7 a sampling pump, 8 an oxidation catalyst unit, 9 (9a and 9b) a NO-NO2 converter unit, 10 a NO analyzer, 11 an ozone decomposition unit, 12 ozone generating means, 13 a changeover valve, 14 arithmetic processing means, and 15 a line (an oxygen adding line), respectively.

### DETAILED DESCRIPTION OF THE INVENTION

Embodiments of the invention will be described with reference to the drawings.

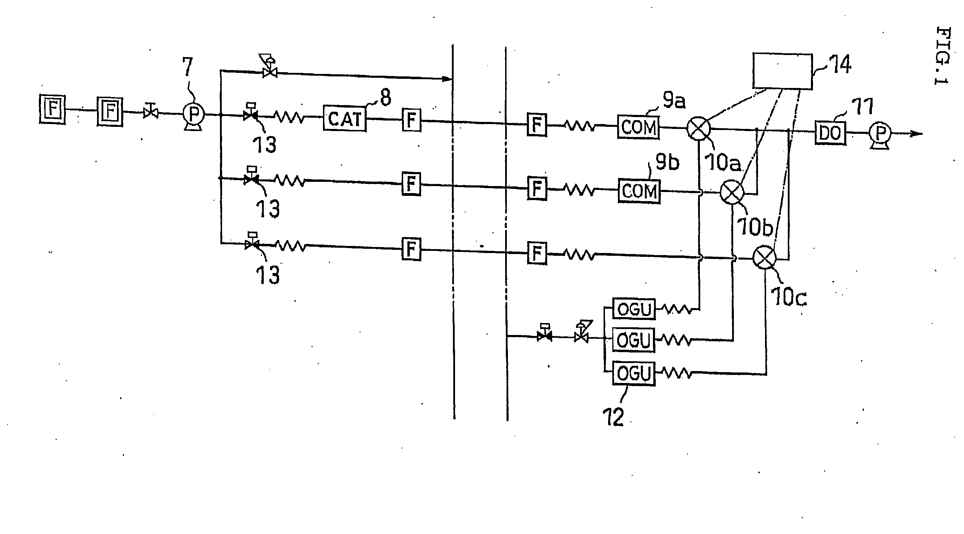

Fig. 1 shows an example of the configuration of the nitrogen compound analyzer according to the invention, which uses a chemiluminescent detector (CLD) as the NO analyzer. A sample is introduced from a sample inlet by means of a sampling pump 7 and divided into three parts (first, second and third parts). The first part is introduced into an NO analyzer 10a through an oxidation catalyst unit 8 (oxidation means for oxidizing ammonia in the sample) and an NO-NO2 converter unit 9a (conversion means for converting nitrogen dioxide in the sample to nitrogen monoxide) and discharged through an ozone decomposition unit 11 (a treatment system (A)). The second part is introduced into an NO analyzer 10b through an NO-NO2 converter unit 9b and discharged through the ozone decomposition unit 11 (a treatment system (B)). The third part is introduced into an NO analyzer 10c as it is and discharged through the ozone decomposition unit 11 (a treatment system (C)). Ozone (03) gas is supplied to the NO analyzers 10 (10a, 10b and 10c) from ozone generating means 12 based on an oxygen source.

In this configuration, the total nitrogen compounds of the sample can be measured in the treatment system (A), NOX in the treatment system (B), and NO in the treatment system (C). Thus, the output of each of the NO analyzers 10a, 10b and 10c is introduced to arithmetic processing means 14, in which the difference between the measurements is calculated so that NH3 (=(A)-(B)) and NO2 (=(B)-(C)) can be measured and the five nitrogen compound components can be continuously measured.

For the purpose of measuring a specific one(s) of the five nitrogen compound components, changeover valves 13 may be provided immediately downstream from the sampling pump 7, and changeover and operation may be performed such that necessary one(s) of the treatment systems is only connected.

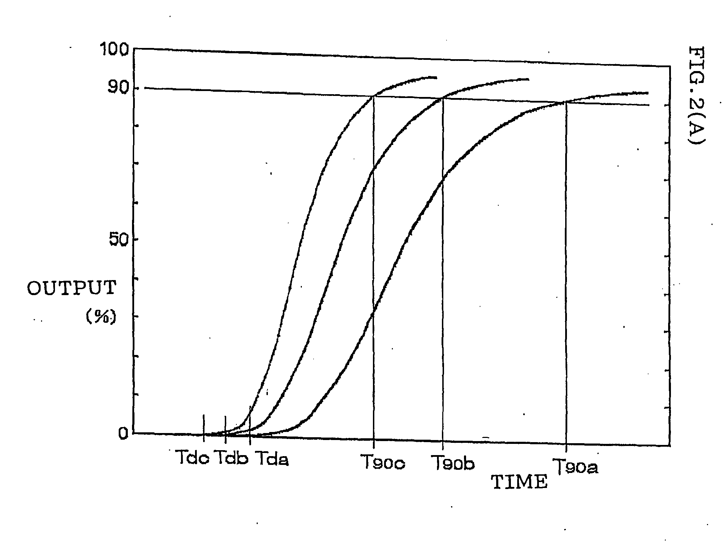

In a preferred mode, the outputs of the NO analyzers 10a, 10b and 10c in the respective treatment systems are each corrected using Td and T90 which are response time information specific to the outputs. For example, as illustrated in Fig. 2(A), when the corresponding component of a sample 10 is introduced into each of the treatment systems (A) to (C), the outputs of the NO analyzers 10a, 10b and 10c each have a specific time lag (Tda, Tdb or Tdc) and a specific 90% response (T90a, T90b or T90c). These are the results of the combination of the responses of the gas system and the signal processing system in the NO analyzers 10a, 10b and 10c and the responses depending on the gas flow rate in each treatment system and depending on the reactivity and the absorption properties in the treatment means such as the oxidation catalyst unit 8. When the flow rates of the respective treatment systems are substantially the same, the relationships Tda>Tdb>Tdc and T90a>T90b>T90c are generally satisfied. These response properties may less change in the respective treatment systems unless the composition extremely fluctuates. If Td and T90 are determined with respect to each treatment system in advance and if the response time information is stored in the arithmetic processing means 14, the outputs of the NO analyzers 10a, 10b and 10c can be corrected during actual measurement.

In a preferred mode, the correction is made first for Td and second for T90. Two methods of the correction will be described in detail below. For the purpose of simplifying the description, outputs from two NO analyzers are used for the description, but it will be understood that the description is not intended to limit the scope of the invention.

### Correction Method 1

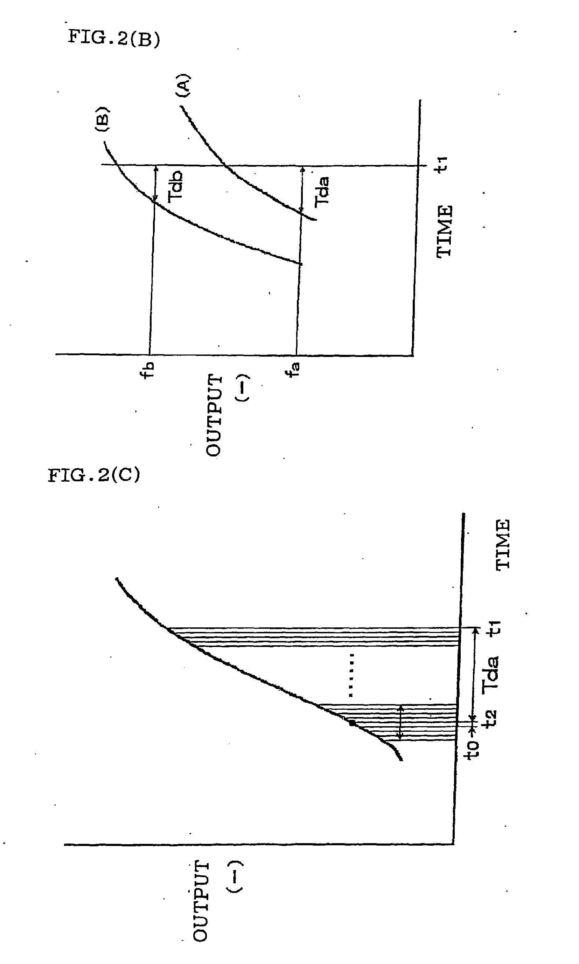

For example, with respect to two treatment systems having the same starting time point, the outputs of the NO analyzers in the treatment systems (A) and (B) are represented by fa and fb, respectively. Corrected outputs fn(A) and fn(B) of the NO analyzers may be calculated according to the following procedure:
(1) Preliminarily, with respect to the change in sample concentration, time lags Tda and Tdb and 90% responses T90a and T90b in the respective treatment systems are obtained and stored in the arithmetic processing means 14.
(2) Reference rise properties are determined, and reference T90s is determined. Typically, referring to the treatment system with the slowest response, the maximum value T90a is selected from T90a and T90b.
(3) During actual measurement, the output of each NO analyzer is stored, and as shown in Fig. 2(B), outputs fa and fb are calculated from the respective outputs at a certain time t1 by shifting the time from t1 by Tda and Tdb, respectively.
(4) The step (3) is sequentially repeated so that outputs can be continuously produced. Arithmetic processing is performed using T90a as a processing time constant for smoothing, zero adjustment or span (sensitivity) adjustment for the outputs fa and fb.
(5) According to the above process, the outputs fa and fb are each corrected for response lag to give outputs fn(A) and fn(B), and the concentration at the time t1 is calculated and output or displayed. Correction Method 2

The outputs of the NO analyzers may further be subjected to moving average processing so that higher correction precision can be achieved. Specifically, corrected outputs may be calculated according to the following procedure:
(1) Preliminarily, with respect to the change in sample concentration, time lags Tda and Tdb and 90% responses T90a and T90b in the respective treatment systems are obtained and stored in the arithmetic processing means 14 having the function of moving average processing.
(2) As illustrated in Fig. 2(C), a moving average unit time t0 is set, and the amounts of shift for moving average corresponding to Tda and Tdb, specifically as many as T90a/t0 for the treatment system (A) and as many as T90b/t0 for the treatment system (B), are stored for calculation. For example, T90a/t0 arithmetic parameter in the low-response treatment system (A) are determined for the moving average mode corresponding to T90.
(3) During actual measurement, the output of each NO analyzer is sequentially stored in the arithmetic processing means 14, and output data corresponding to the arithmetic parameter are averaged based on the respective output values at the times t2 shifted from t1 by T90a and T90b, respectively, so that calculated and corrected outputs fn(A) and fn(B) are obtained. In this process, the moving average method for each output value at the time t1 may be a method including the step of calculating the average of the output data corresponding to the arithmetic parameter with the center at the time t2, a method including the step of calculating the average of the output data corresponding to the arithmetic parameter by the time t2, or a method including the step of calculating the average of the output data corresponding to the arithmetic parameter at and after the time t2.
(4) The steps (2) and (3) are repeated every unit time t0 so that calculated outputs can be continuously obtained.
(5) According to the above process, the outputs fa and fb are corrected for response lag, and the concentration is calculated and output or displayed.

As described above, Td and T90 are preliminarily obtained for each treatment system so that the outputs of the NO analyzers 10a, 10b and 10c can be properly corrected during actual measurement.

Particularly in the moving average processing method, time lag or arithmetic parameter can be set easily and freely, and actual measurements can be very effectively corrected for response lag. For example, the moving average processing method is effective in the following cases:
(1) In the case of measurement of components in automobile exhaust gas, the amounts of discharged NH3 or NO2 significantly differ between a Diesel engine and a gasoline engine, and even with the same sample treatment system, the response properties can significantly vary depending on the reaction or adsorption in the flow channel and the influence of coexisting water or particulates. In terms of response, such properties can be classified into some patterns by the type of engine or by the type of exhaust gas treatment in automobiles. Thus, different moving average modes according to such measurement conditions may be stored in the arithmetic processing means 14 so that the measurement precision can be improved, for example, by inputting information on the vehicle type or the like and changing the moving average modes.
(2) It is also known that the response properties of sample treatment systems may vary depending on the concentration of NH3 or NO2 in samples. For example, the 90% response rises as the concentration decreases, or it falls as the concentration increases. In the analyzer, concentration ranges for measurement (for example, 0-10/0-100/0-1,000 ppm for measurement of NH3) may be selected at any time depending on the amount of discharged NH3 or NO2 in a sample. Moving average modes may be switched depending on the selected concentration range for measurement so that a correction can be accurately made for response lag.
(3) If relatively stable concentrations are provided in a certain time zone, in some cases, it may be useful to switch moving average modes depending on actual measurement. Thus, the changeover in moving average modes depending on the concentration range for measurement may be sophisticated by preliminarily making a data base of an association table of the outputs of the analyzers and moving average modes so that the arithmetic processing means 14 can automatically make a decision based on the outputs of the analyzers and can allow the changeover in the moving average modes. Thus, a correction can be accurately made for response lag.

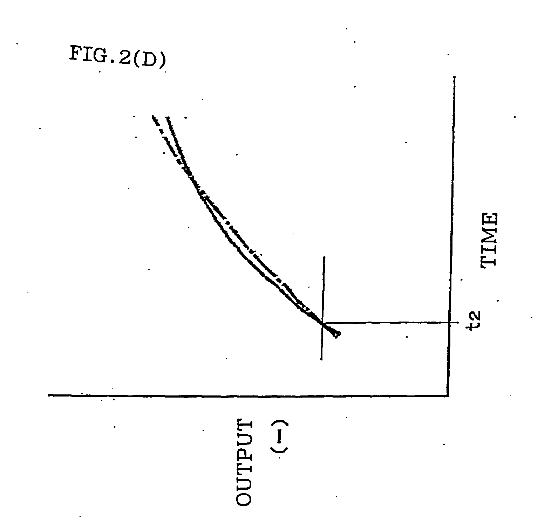

Without using the simple moving average of the arithmetic parameter of the analyzer outputs, the moving average processing method according to the invention may include the steps of: dividing the arithmetic parameter into plural parts; combining the plural moving average modes to form a new moving average mode; and multiplying it by the output of the analyzer, so that the accuracy can be increased. If the concentration is relatively low, for example, low-order curve approximation such as second- or third-order approximation would be enough for the response curve using time as parameter. If the concentration is relatively high, however, such approximation would have a large deviation. The response curve may also vary with the influence of a change in reaction efficiency in the treatment means. For example, the response curve may vary as shown in Fig. 2(D) even after a correction is made for time lag. In such cases, different moving average modes may be combined so that the accuracy can further be improved. It will be understood that the invention may also use any moving average processing method in addition to the above methods and any combination of the above methods.

Independent moving average processing means may be provided separately from the arithmetic processing means 14, and the changeover in the moving average modes or the change of the arithmetic parameter may be manually or automatically performed. The input function may be provided inside the analyzer or outside the analyzer.

The oxidation catalyst unit 8 has a platinum-based catalyst and/or a nickel-based catalyst in its interior and can oxidize ammonia in the sample under the conditions of about 800 to 900°C. The NO-NO2 converter unit 9 has a carbon-based catalyst in its interior and can reduce and convert NO2 in the sample to NO under the conditions of about 150 to 250°C. The ozone decomposition unit 11 can thermally decompose 03 in the sample under the conditions of about 200 to 300°C to make the exhaust gas harmless.

The above-described structure specifically uses a platinum-based catalyst and/or a nickel-based catalyst as an NH3-oxidizing catalyst but may alternatively use any other oxidation catalyst such as a palladium-based catalyst and a copper-or chromium-based catalyst. The platinum-based catalyst and/or the nickel-based catalyst is preferably used in order to form a mesh structure and to provide the desired oxidation efficiency (for example, a conversion efficiency of 90%) or higher.

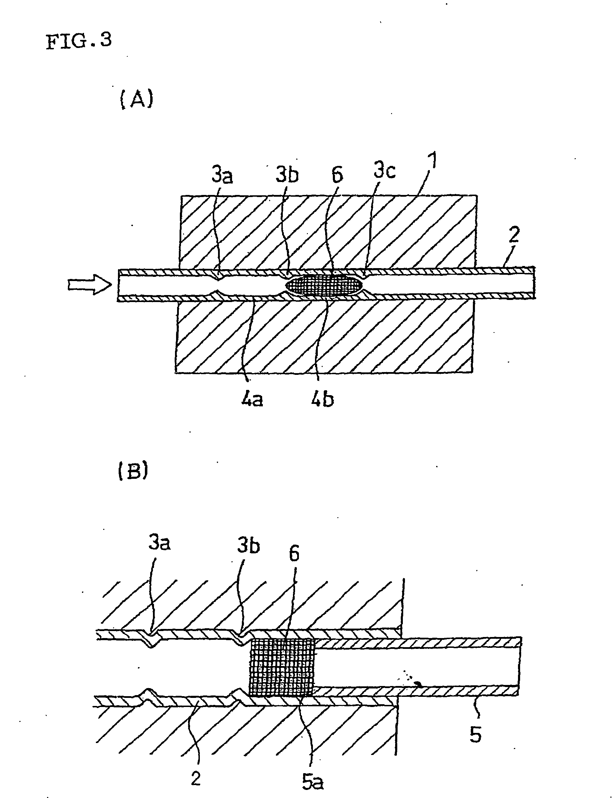

Each treatment means such as the oxidation catalyst unit 8 preferably has the structure as illustrated in Fig. 3(A). A tubular part 2 that forms a flow channel is placed inside a heating part 1. The tubular part 2 has projection portions 3 (specifically 3a, 3b and 3c) at different sites and parallel and flat-shaped portions 4 (specifically 4a and 4b) upstream and downstream of the projection portion 3 in the flow channel so that it can quickly heat the sample and raise the temperature of the sample. If a catalyst 6 is placed in the parallel and flat-shaped part 4b between the projection portions 3b and 3c, the sample is heated at the projection portion 3a and then at the portion 3b so that the catalyst 6 can function under conditions sufficiently heated to a specific temperature. As illustrated in Fig. 3(B), another tubular part 5 with a different diameter may be inserted in the tubular part 2. The tubular part 5 inserted in the tubular part 2 has an outside diameter substantially the same as the inside diameter of the tubular part 2. The end portion 5a of the tubular part 5 has the same function as the projection portion 3c as shown in Fig. 3(A) and increases the gas flow rate after the heating so that it can form an efficient heating unit. Such heating means or such a catalyst unit may be used to form the oxidation catalyst unit 8, the NO-NO2 converter unit 9 or the ozone decomposition unit 11 and can significantly contribute to making the whole system compact. Such heating means or such a catalyst unit may also be used under various temperature conditions. In particular, it is very superior because it can have substantially the same function under high temperature conditions at 800°C or higher.

The catalyst 6 may have any selected shape and composition depending on purpose when installed in the catalyst unit. If the catalyst is fixed by means of the projection portions 3b and 3c as shown in Fig. 3(A), the catalyst should preferably have a mesh shape. The mesh-shaped catalyst 6 can be free from an increase in pressure loss and can be fixed without using a filter or a holder member, so that adsorption-induced loss or response delay can be prevented. The catalyst 6 is also superior because it can be easily attached or detached and easily replaced.

As described above, according to the configuration of the invention, a device for analyzing nitrogen compounds including NH3 and NO2 can be constructed which can show less adsorption of NH3 or NO2 and can show very high responsiveness by making a quick conversion from NH3 to NOx. Pressure loss can be prevented in the NH3 → NOx conversion flow channel, and adsorption or loss of any other component in the sample can also be prevented, so that a response difference can hardly occur between the flow channels with a treatment process and with no treatment process. Even if a slight response lag is generated, measurement can be performed with high precision with no influence of response lag by the correction according to the above method, because each flow channel has independent response properties.

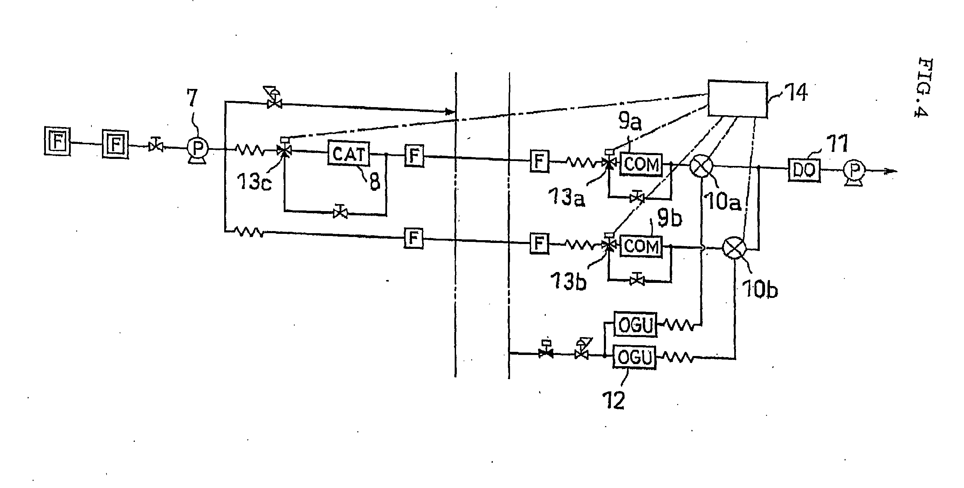

Fig. 4 shows another example of the configuration of the analyzer according to the invention, in which the functions corresponding to the above treatment systems (A) to (C) can be provided by switching changeover valves 13 (13a, 13b and 13c) each placed upstream from each treatment means, and each of five nitrogen compound components can be easily and accurately measured using two branch flow channels. The states of the changeover valves 13 and treatment modes including measuring objects in this configuration are summarized in Table 1.

**Table 1**

| **Treatment Modes** | **Changeover Valves** | | | **Measuring Components** |
|---|---|---|---|---|
| | 13c | 13a | 13b | |
| A' | OFF | OFF | - | **Nitrogen Compound** |
| B' | - | - | OFF | NOx |
| B" | ON | OFF | - | NOx |
| C' | - | - | ON | NO |
| C" | ON | ON | - | NO |
| A'-B' | OFF | OFF | OFF | NH3 |
| B"-C' | ON | OFF | ON | NO2 |
| B'-C" | ON | ON | OFF | NO2 |

Table 1 indicates that treatment modes can be switched by the control using the changeover valves 13 so that the five components: total nitrogen compounds, NH3, NOx, NO2 and NO can be accurately measured.

As illustrated in Figs. 4 to 6, the changeover valves 13 are each a three-way electromagnetic valve, which forms a straight flow channel at OFF state (connected to OUTLET in white) and forms a straight flow channel at ON state (connected to OUTLET in black). The changeover valves 13a, 13b and 13c do not have to be provided for all of the oxidation catalyst unit 8 and the NO-NO2 converter unit 9a, and any of the valves may be omitted depending on the treatment mode or the arrangement of the units so that the device can be simplified. Specifically, for example, in the device configuration as shown in Figs. 4 to 6, the combination of (A'), (B') and (C") may be selected as the treatment mode shown in Table 1 so that five components can be measured without the changeover valve 13b.

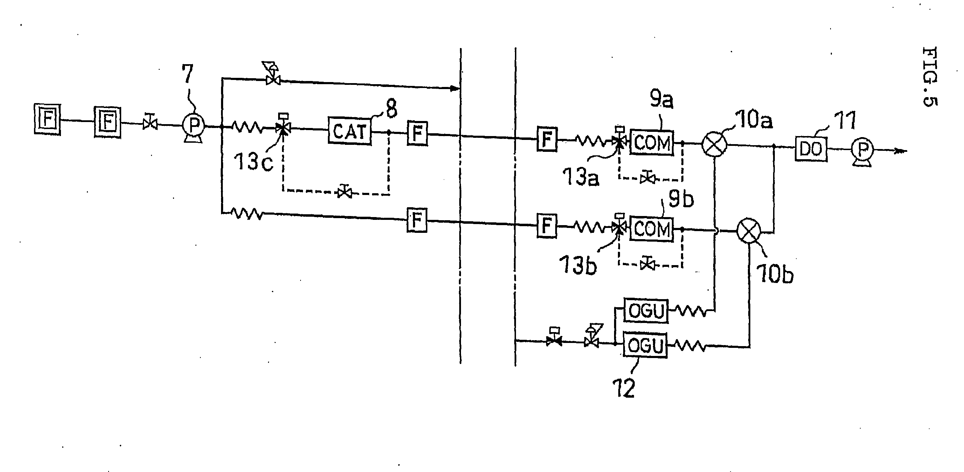

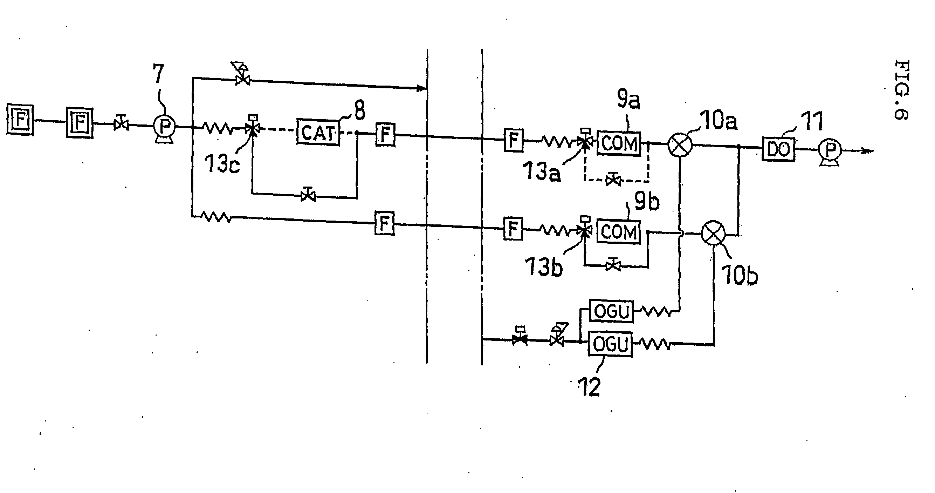

Referring to Figs. 5 and 6, the mechanism of the example as shown in Fig. 4 is specifically described below. For example, when NH3 in a sample is measured as illustrated in Fig. 5, the sample is introduced from a sample inlet by means of a sampling pump 7 and divided into two parts (first and second parts), and (A') the first part is introduced into a NO analyzer 10a through an oxidation catalyst unit 8 and a NO-NO2 converter unit 9a and discharged through an ozone decomposition unit 11, and (B') the second part is introduced into a NO analyzer 10b through a NO-NO2 converter unit 9b as it is and discharged through the ozone decomposition unit 11. In this process, total nitrogen compounds in the sample are measured in the mode (A'), and NOx is measured in the mode (B'), so that NH3 (=(A')-(B')) can be continuously measured.

As illustrated in Fig. 6, (B") when the changeover valve 13c is activated such that the oxidation catalyst unit 8 is bypassed, the sample is introduced into the NO analyzer 10a through the NO-NO2 converter unit 9a and discharged through the ozone decomposition unit 11. (C') When the changeover valve 13b, which is placed immediately upstream from the NO-NO2 converter unit 9b and at which the sample flow channel is branched, is activated such that the unit 9b is bypassed, the sample is introduced into the NO analyzer 10b and discharged through the ozone decomposition unit 11. In this process, NOx in the sample is measured in the mode (B"), and NO is measured in the mode (C'), so that NO2 (=(B")-(C')) can be measured.

(C") When the changeover valve 13c is activated such that the oxidation catalyst unit 8 is bypassed, the changeover valve 13a is activated such that the sample is introduced into the NO analyzer 10a through the NO-NO2 converter unit 9a and discharged through the ozone decomposition unit 11. In this mode, NO can be measured. This mode is used in combination with the treatment mode (B') of simultaneous measurement of NOx so that NO2 (=(C")-(B')) can be measured.

In this process, the outputs of the NO analyzers 10a and 10b are input into the arithmetic processing means 14 in which the difference between the measurements is calculated. The arithmetic processing means 14 also controls the operation of each of the changeover valves 13a, 13b and 13c depending on each treatment mode. At the same time, the correction as described above may be performed depending on each treatment mode so that accurate measurements can be obtained for NH3 and NO2 from the differential outputs.

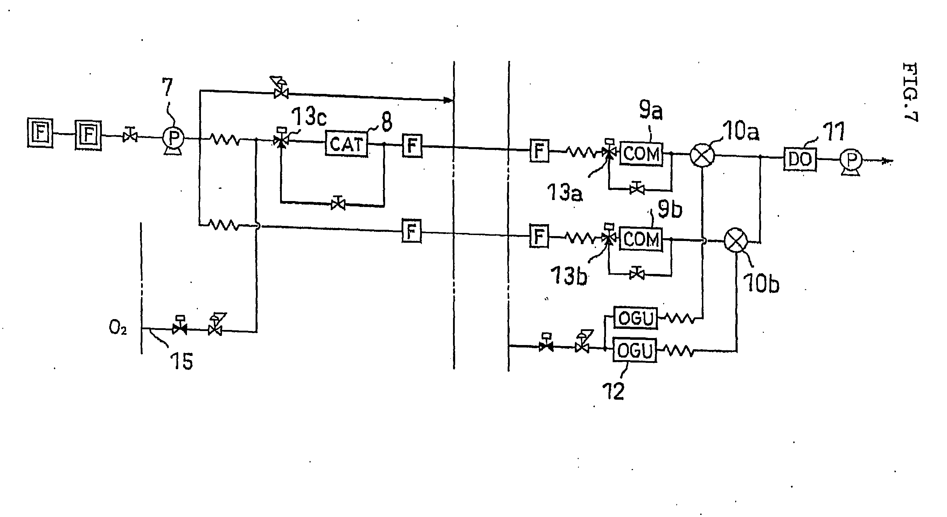

Fig. 7 shows a third example of the configuration. If the sample does not contain sufficient oxygen necessary for oxidation, a specific amount of oxygen (air) may be added from a line 15 to a portion immediately upstream of the oxidation catalyst unit 8 to supplement the combustion-supporting agent in the sample so that high oxidation efficiency can be achieved. Particularly in a case where several thousands ppm of NH3 is momentarily discharged, such as in the case of lean-burn combustion as mentioned above, it should be difficult for the oxygen in the sample to cause sufficient oxidation, and thus the addition of oxygen should preferably be controlled depending on the service conditions of the analyzer. Specifically, about 50 to 100 mL/min of pure oxygen is preferably added to a sample containing several thousands ppm of NH3 at a flow rate of 1 to 3 L/min for the introduction into the oxidation catalyst unit 8.

According to the invention, therefore, there can be provided very suitable means for rapidly measuring NH3 or NOx in automobile exhaust gas. The invention is also applicable to measurement of NH3 or NOx in various types of exhaust gas, for example, including exhaust gas from stationary exhaust sources such as various combustion furnaces.

According to the invention, a wide range of heating temperatures can be covered. Thus, the invention is applicable not only to exhaust gas but also to various processes and studies and, for example, applicable to the following fields:
(1) a single heating unit of a gas-generation simulator for a catalyst evaluation system; and
(2) devices for analyzing the behavior of heated gas in an engine.

## Claims

1. Nitrogen compound analyzer, comprising:
- a plurality of sample treatment systems (A, B, C) by which a single sample is divided, the plurality of sample treatment systems (A, B, C) including:
- a first treatment system (A) having oxidation means (8) for oxidizing ammonia in the sample and conversion means (9a) for converting nitrogen dioxide in the sample to nitrogen monoxide.
- a second treatment system (B) having conversion means (9b) for converting nitrogen dioxide in the sample to nitrogen monoxide, and
- a third treatment system (C) in which no conversion treatment is performed on a specific component in the sample.
- a changeover means (13) capable of providing a specific combination of any of the treatment systems (A, B, C),
- at least one measurement means (10a, 10b, 10c) for continuously measuring nitrogen monoxide in the sample,
- an arithmetic processing means (14).
- wherein said arithmetic processing means (14) is configured for operating the output of the measurement means (10a, 10b, 10c), and
- **characterized by** said arithmetic processing means (14) being configured for correcting the output of the measurement means (10a, 10b, 10c) with the information preliminarily obtained, which is specific to each sample treatment system (A, B, C) and comprises time lags Td and x% responses Tx in the respective treatment systems (A, B, C).

2. Nitrogen compound analyzer according to claim 1.
which is configured in order to provide a correcting operation of the arithmetic processing means according to the following procedure:
(1) obtaining and storing in the arithmetic processing means preliminarily, with respect to the change in sample concentration, time lags Td and Tx in the respective treatment systems.
(2) determining a reference Tx in the respective treatment systems,
(3) during actual measurement, the storing output of the measurement means in the respective treatment systems, and calculating outputs from the respective outputs at an arbitrary time by shifting the time from the arbitrary time by Td, respectively,
(4) wherein step (3) is sequentially repeated continuously at the arbitrary time so that outputs can be continuously produced.
(5) calculating the concentration at the arbitrary time by using the outputs corrected each for response lag in the respective treatment systems, and outputing or displaying said outputs.

3. Nitrogen compound analyzer according to claim 1, further comprising a correcting operation of the arithmetic processing means according to the following procedure;
(1) obtaining and storing in the arithmetic processing means preliminarily, with respect to the change in sample concentration, time lags Td and Tx in the respective treatment systems,
(2) setting a moving average unit time t0, storing for calculation the amounts of shift for moving average corresponding to Td, specifically as many as Td/t0 for the respective treatment system, are, and determining Tx/t0 arithmetic parameter in the respective treatment system for the moving average mode corresponding to Tx.
(3) during actual measurement, storing sequentially in the arithmetic processing means the output of the measurement means in the respective treatment systems, and averaging output data corresponding to the arithmetic parameter based on the respective output values at the time shifted from the arbitrary time by Tx, so that calculated and corrected outputs are obtained in the respective treatment systems.
(4) wherein steps (2) and (3) are repeated every unit time t0 so that calculated outputs can be continuously obtained.
(5) calculating the concentration at the arbitrary time by using the outputs corrected each for response lag in the respective treatment systems, and outputing or displaying said outputs.

4. Nitrogen compound analyzer according to any one of the preceding claims,
wherein the oxidation means (8) or the conversion means (9a, 9b) comprise:
- a flow channel (2) formed inside a heating part (1),
- at least one projection portion or step portion (3a,3b,3c) provided in the flow channel.
- a flat inner surface region (4a,4b) of a specific length, which is parallel to the centerline of the flow channel and provided upstream and downstream of the projection or step portion in the flow channel, and
- the oxidation catalyst or the conversion catalyst (6) set on the other flat region of the upstream and downstream of the projection or step portion.

## Patentansprüche

1. Stickstoffverbindungsanalysator,
mit
- einer Mehrzahl Probenbearbeitungssysteme (A, B, C), durch welche eine einzelne Probe aufgeteilt wird, wobei die Mehrzahl Probenbearbeitungssysteme (A, B, C) aufweist:
- ein erstes Bearbeitungssystem (A) mit einer Oxidationseinrichtung (8) zum Oxidieren von Ammoniak in der Probe und eine Umwandlungseinrichtung (9a) zum Umwandeln von Stickstoffdioxid in der Probe in Stickstoffmonoxid,
- ein zweites Bearbeitungssystem (B) mit einer Umwandlungseinrichtung (9b) zum Umwandeln von Stickstoffdioxid in der Probe in Stickstoffmonoxid und
- ein drittes Bearbeitungssystem (C), bei welchem keine Umwandlungsbearbeitung in Bezug auf eine spezifische Komponente in der Probe durchgeführt wird,
- einer Umschalteinrichtung (13), die in der Lage ist, eine bestimmte Kombination der Bearbeitungssysteme (A, B, C) bereitzustellen,
- mindestens einer Messeinrichtung (10a, 10b, 10c) zum kontinuierlichen Messen von Stickstoffmonoxid in der Probe und
- einer arithmetischen Verarbeitungseinrichtung (14),
- wobei die arithmetische Verarbeitungseinrichtung (14) ausgebildet ist, die Ausgabe der Messeinrichtungen (10a, 10b, 10c) zu verarbeiten,
- **dadurch gekennzeichnet, dass** die arithmetische Verarbeitungseinrichtung (14) ausgebildet ist, die Ausgabe der Messeinrichtungen (10a, 10b, 10c) zu korrigieren, und zwar in Bezug auf zuvor erhaltene Information, welche spezifisch ist für jedes der Probenbearbeitungssysteme (A, B, C) und welche Zeitverzögerungen Td und x%-Antworten Tx in den jeweiligen Bearbeitungssystemen (A, B, C) aufweist.

2. Stickstoffverbindungsanalysator nach Anspruch 1,
welcher ausgebildet ist, einen Korrekturvorgang in Bezug auf die arithmetische Verarbeitungseinrichtung bereitzustellen, und zwar gemäß dem nachfolgenden Ablauf:
(1) Erhalten und Speichern der Zeitverzögerungen Td und Tx in den jeweiligen Bearbeitungssystemen in der arithmetischen Verarbeitungseinrichtung in vorläufiger Art und Weise in Bezug auf die Änderung der Probenkonzentration.
(2) Bestimmen eines Referenzwerts Tx in den jeweiligen Bearbeitungssystemen,
(3) während der tatsächlichen Messung, Speichern der Ausgabe der Messeinrichtungen in den jeweiligen Bearbeitungssystemen und Berechnen von Ausgaben aus den jeweiligen Ausgaben zu einer beliebigen Zeit durch Verschieben der Zeit von beliebigen Zeit um jeweils Td,
(4) wobei der Schritt (3) aufeinanderfolgend kontinuierlich zu der beliebigen Zeit wiederholt wird, so dass Ausgaben kontinuierlich erzeugt werden können,
(5) Berechnen der Konzentration zu der beliebigen Zeit durch Verwenden der Ausgaben, die jeweils korrigiert wurden im Hinblick auf die Antwortverzögerung in den jeweiligen Bearbeitungssystemen, und Ausgeben oder anzeigen der Ausgaben.

3. Stickstoffverbindungsanalysator nach Anspruch 1, des Weiteren aufweisend einen Korrekturvorgang der arithmetischen Verarbeitungseinrichtung gemäß dem nachfolgenden Ablauf:
(1) Erhalten und Speichern der Zeitverzögerungen Td und Tx in den jeweiligen Bearbeitungssystemen in der arithmetischen Verarbeitungseinrichtung in vorläufiger Art und Weise im Hinblick auf die Änderung der Probenkonzentration,
(2) Einstellen einer gleitenden Mittelungszeiteinheit t0, Speichern zur Berechnung der Werte der Verschiebung zur gleitenden Mittelung korrespondierend zum Wert Td, insbesondere in einer Anzahl korrespondierend zu Td/t0 für das jeweilige Bearbeitungssystem, und bestimmen eines Tx/t0 arithmetischen Parameters im jeweiligen Bearbeitungssystem für den Modus mit gleitendem Mittelwert korrespondierend zu dem Wert Tx,
(3) während der tatsächlichen Messung aufeinander folgendes Speichern der jeweiligen Ausgabe der Messeinrichtungen in den jeweiligen Bearbeitungssystemen in der arithmetischen Verarbeitungseinrichtung und Mitteln der Ausgabedaten korrespondierend zum arithmetischen Parameter auf der Grundlage der jeweiligen Ausgabewerte zu einer Zeit, die von der beliebigen Zeit um Tx verschoben ist, so dass berechnete und korrigierte Ausgaben in dem jeweiligen Bearbeitungssystemen erhalten werden,
(4) wobei die Schritte (2) und (3) jede Zeiteinheit t0 derart wiederholt werden, dass die berechneten Ausgaben kontinuierlich erhalten werden können,
(5) Berechnen der Konzentration zu der beliebigen Zeit durch Verwenden der Ausgaben, die in Bezug auf jede Antwortverzögerung in den jeweiligen Bearbeitungssystemen korrigiert sind, und Ausgeben oder Anzeigen der Ausgaben.

4. Stickstoffverbindungsanalysator nach einem der vorangehenden Ansprüche,
bei welchem die Oxidationseinrichtung (8) oder die Umwandlungseinrichtungen (9a, 9b) aufweisen:
- einen Fließkanal (2), der im Inneren eines Heizbereichs (1) ausgebildet ist,
- mindestens einen hervorstehenden Bereich oder Stufenbereich (3a, 3b, 3c), welche im Fließkanal ausgebildet sind,
- einen Bereich (4a, 4b) mit flachen innerer Fläche einer bestimmten Länge, welcher parallel ausgebildet ist zur Mittellinie des Fließkanals und welcher stromaufwärts und stromabwärts vom hervorstehenden Bereich oder Stufenbereich im Fließkanal ausgebildet ist, und
- den Oxidationskatalysator oder Umwandlungskatalysator (6), der vorgesehen ist im anderen flachen Bereich stromaufwärts und stromabwärts in Bezug auf den hervorstehenden Bereich oder Stufenbereich.

## Revendications

1. Analyseur de composé azoté,
comprenant :
- plusieurs systèmes de traitement d'échantillon (A, B, C) par lesquels un échantillon unique est divisé, les plusieurs systèmes de traitement d'échantillon (A, B, C) comprenant :
- un premier système de traitement (A) ayant un moyen d'oxydation (8) pour oxyder l'ammoniac dans l'échantillon et un moyen de transformation (9a) pour transformer le dioxyde d'azote dans l'échantillon en monoxyde d'azote,
- un deuxième système de traitement (B) ayant un moyen de transformation (9b) pour transformer le dioxyde d'azote dans l'échantillon en monoxyde d'azote, et
- un troisième système de traitement (C) dans lequel aucun traitement de transformation n'est effectué sur un composant spécifique dans l'échantillon,
- un moyen de changement (13) susceptible de fournir une combinaison spécifique de n'importe lesquels des systèmes de traitement (A, B, C),
- au moins un moyen de mesure (10a, 10b, 10c) pour mesurer continuellement le monoxyde d'azote dans l'échantillon,
- un moyen de traitement arithmétique (14),
- dans lequel ledit moyen de traitement arithmétique (14) est configuré pour mettre en oeuvre la sortie des moyens de mesure (10a, 10b, 10c), et
**caractérisé en ce que** ledit moyen de traitement arithmétique (14) est configuré pour corriger la sortie des moyens de mesure (10a, 10b, 10c) avec les informations préalablement obtenues, qui sont spécifiques à chaque système de traitement d'échantillon (A, B, C) et qui comprennent des retards de temps Td et des réponses Tx à x % dans les systèmes de traitement respectifs (A, B, C).

2. Analyseur de composé azoté selon la revendication 1,
qui est configuré pour réaliser une opération de correction du moyen de traitement arithmétique selon la procédure suivante :
(1) obtention et stockage dans le moyen de traitement arithmétique, préalablement, en ce qui concerne le changement de concentration d'échantillon, de retards de temps Td et Tx dans les systèmes de traitement respectifs,
(2) détermination d'une référence Tx dans les systèmes de traitement respectifs,
(3) pendant la mesure réelle, stockage de la sortie du moyen de mesure dans les systèmes de traitement respectifs, et calcul des sorties à partir des sorties respectives à un instant arbitraire en décalant le temps de Td par rapport au temps arbitraire, respectivement,
(4) où l'étape (3) est répétée continuellement de manière séquentielle à l'instant arbitraire de sorte que des sorties peuvent être continuellement produites,
(5) calcul de la concentration à l'instant arbitraire en utilisant les sorties, chacune étant corrigée en ce qui concerne le retard de réponse dans les systèmes de traitement respectifs, et sortie ou affichage desdites sorties.

3. Analyseur de composé azoté selon la revendication 1, comprenant en outre une opération de correction du moyen de traitement arithmétique selon la procédure suivante :
(1) obtention et stockage dans le moyen de traitement arithmétique, préalablement, en ce qui concerne le changement de la concentration d'échantillon, de temps de retard Td et Tx dans les systèmes de traitement respectifs,
(2) fixation d'une unité de temps moyenne mobile t0, stockage pour calcul des quantités de décalage pour la moyenne mobile correspondant à Td, de manière spécifique autant qu'il y a de Td/t0 pour le système de traitement respectif, et détermination du paramètre arithmétique Tx/t0 dans le système de traitement respectif pour le mode de moyenne mobile correspondant à Tx,
(3) pendant la mesure réelle, stockage séquentiel dans le moyen de traitement arithmétique de la sortie du moyen de mesure dans les systèmes de traitement respectifs, et calcul de la moyenne des données de sortie correspondant au paramètre arithmétique en se basant sur les valeurs de sortie respectives à l'instant décalé de Tx par rapport au temps arbitraire, de sorte que l'on obtient des sorties calculées et corrigées dans les systèmes de traitement respectifs,
(4) où les étapes (2) et (3) sont répétées à chaque unité de temps t0 de sorte que l'on peut continuellement obtenir des sorties calculées,
(5) calcul de la concentration à l'instant arbitraire en utilisant les sorties, chacune étant corrigée en ce qui concerne le retard de réponse dans les systèmes de traitement respectifs, et sortie ou affichage desdites sorties.

4. Analyseur de composé azoté selon l'une quelconque des revendications précédentes,
dans lequel le moyen d'oxydation (8) ou les moyens de transformation (9a, 9b) comprennent :
- un canal d'écoulement (2) formé à l'intérieur d'un élément chauffant (1),
- au moins une partie en saillie ou une partie étagée (3a, 3b, 3c) disposée dans le canal d'écoulement,
- une zone superficielle intérieure plate (4a, 4b) d'une longueur spécifique, qui est parallèle à la ligne médiane du canal d'écoulement et disposée en amont et en aval de la partie en saillie ou de la partie étagée dans le canal d'écoulement, et
- un catalyseur d'oxydation ou un catalyseur de transformation (6) positionné sur l'autre zone plate en amont et en aval de la partie en saillie ou de la partie étagée.
